# EUROPEAN PATENT APPLICATION

(11) **EP 3 960 748 A1**
(43) Date of publication of application: **02.03.2022**
(21) Application number: 20796375.2
(22) Date of filing: 27.04.2020
(51) Int. Cl.: C07H 1/08, C07D 231/40, A61K 8/30, A61K 31/7048, A61Q 19/00

(54) **COMPOSITION FOR THE TREATMENT OF THE SIDE EFFECTS OF RADIOTHERAPY AND/OR CHEMOTHERAPY**

(30) Priority: 25.04.2019 ES 201930367
(71) Applicant: Fundación Profesor Novoa Santos, 15006 A Coruna (ES); Servizo Galego de Saúde (SERGAS), 15703 Santiago de Composteia (ES); Universidade Da Coruña, 15001 A Coruña (ES); Fundación para la Investigación Biomédica del Hospital Universitario Ramón y Cajal, 28034 Madrid (ES)
(72) Inventor: MAYÁN SANTOS, María Dolores, 15006 A Coruña (ES); ACEA NEBRIL, Benigno, 15006 A Coruña (ES); VARELA EIRÍN, Marta, 15006 A Coruña (ES); RODRÍGUEZ CANDELA MATEOS, Marina, 15006 A Coruña (ES); DÍAZ CARBALLA, Carlota Czestokowa, 15006 A Coruña (ES); FONSECA CAPDEVILA, Eduardo, 15006 A Coruña (ES); BARRIO CALVO, Luis Carlos, 28034 Madrid (ES); ESCUDERO LÓPEZ, Adela, 28034 Madrid (ES)
(74) Representative: Hoffmann Eitle
(86) International application number: PCT/ES2020/070269
(87) International publication number: WO 2020/216984

(57) **Abstract**

The present invention relates to the use of a composition for the treatment and/or prevention of the side-effects of radiotherapy and/or chemotherapy, preferably as a protective post-treatment composition, that is, the effect is produced once the cells have been radiated (restores phenotype and gap junctions and reduces senescence). Accordingly, the composition comprises two chemical compounds that can protect and treat tissues that have been affected or are at risk of being affected by the side effects of chemotherapy and/or radiotherapy. The invention also relates to a kit comprising the composition of the invention for the treatment and/or prevention of the side-effects of radiotherapy and/or chemotherapy.

## Description

### Technical Field

The present invention is comprised in the field of biomedicine. In particular, the present invention provides a composition for the treatment and/or prevention of the side effects of radiotherapy and/or chemotherapy in an animal or individual. The present invention also provides a kit for the treatment and/or prevention of the side effects of radiotherapy and/or chemotherapy in an animal or individual.

### Prior Art

Oleuropein is a chemical compound which can be extracted from green olive pulp and olive tree leaves of the *Olea europaea* variety. Oleuropein is the main phenolic component of green olive pulp and can also be found in extra virgin olive oil, giving it its bitter flavor.

It has been demonstrated that oleuropein is an active compound with antioxidant, antiinflammatory, antiatherogenic, anticancer, antimicrobial, and antiviral properties (Omar, 2010, Sci Pharm 78 (2): 133-54). It has also been demonstrated that oleuropein can be used for treating cutaneous scars or rough or aged skins (US 8,591,961) and for improving skin healing after a cutaneous incision Mehraein, et al., 2014, Cell J. 16 (1): 25-30).

Breast cancer affects about 12% of women worldwide (McGuire et al., 2015, Cancers 7 (2): 908-29). Common methods for treating breast cancer include radiotherapy and chemotherapy. Radiotherapy and chemotherapy can damage the skin, causing, by way of example, radiation-induced fibrosis, inflammation, and dermatitis. Therefore, there is a need to provide compositions capable of preventing, curing, and/or palliating the side effects of radiotherapy and/or chemotherapy.

The authors of the present invention have developed a composition for use in the treatment (preferably as a protective post-treatment composition, since the effect is produced once the cells have been radiated, restoring phenotype and intercellular communication by means of gap junctions and reducing senescence) or prevention of the side effects caused by radiotherapy and/or chemotherapy and a kit comprising said composition for the same use. Specifically, the present invention restores the phenotype of the dermal fibroblast, reduces the degree of cellular senescence, and inhibits the post-treatment synthesis of SASP (senescence-associated secretory phenotype) component to promote tissue regeneration and prevent fibrosis and other degenerative effects.

### Description of the Figures

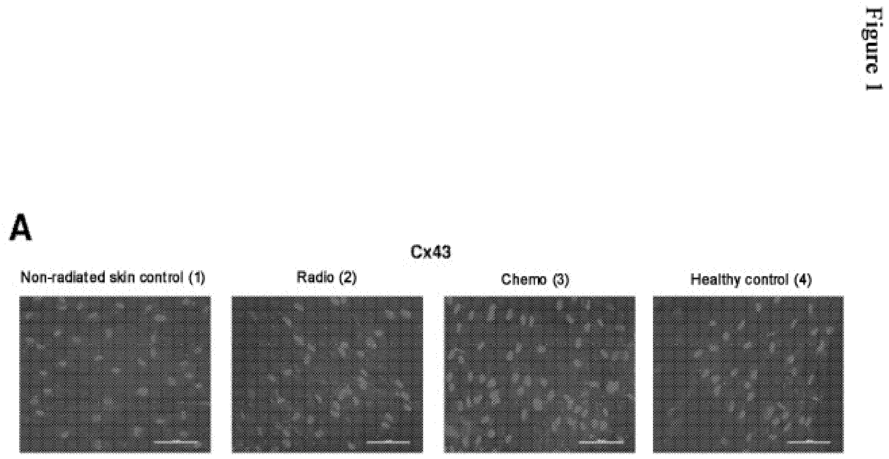

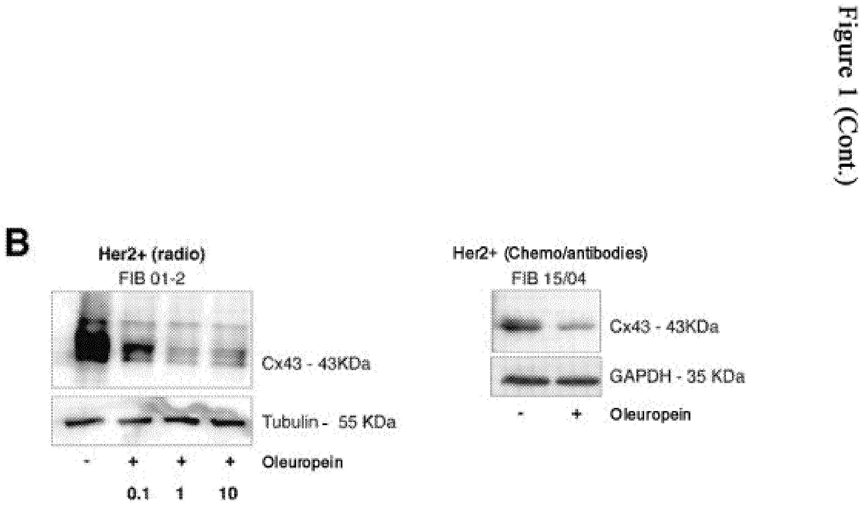

Figure 1. Increase in levels of connexin43 (Cx43) in dermal fibroblasts of breast cancer patients subjected to chemotherapy or radiotherapy. A. Immunofluorescence for the detection of Cx43 (in red), in which there can be seen an increase in Cx43 in dermal fibroblasts isolated from the skin of patients with different subtypes of breast cancer and who have been treated with radiotherapy or chemotherapy in comparison with fibroblasts of patients with a benign pathology (not subjected to treatment) (healthy control, 4). The cell nuclei stained with DAPI are seen in blue. Sample 1 (skin from the back) and sample 2 (skin in the breast region) correspond with fibroblasts isolated from two skin regions of the same patient with Her2+ breast cancer. Sample 1 has not been subjected to radiotherapy, and the patient had been treated with chemotherapy and anti-Her2 antibodies one year before obtaining the skin sample. Sample 2 underwent radiotherapy before it was obtained. Sample 3 corresponds to the skin of a patient with luminal B Her2- breast cancer subjected to neoadjuvant chemotherapy six weeks before taking the sample. Sample 4 comes from a patient with a benign disease and not subjected to any treatment. B. Treatment with different concentrations of oleuropein (0.1 µM, 1 µM, and 10 µM for 2 hours) of fibroblasts isolated from a donor with Her2+ breast cancer reduced the levels of Cx43 detected in the fibroblasts isolated from the skin samples which have been subjected to radiotherapy (left) or to chemotherapy (right). To perform these assays, the same number of cells was used, and tubulin or GAPDH were used as loading control in Western blot. In all cases, oleuropein reduced the levels of Cx43 both in dermal fibroblasts isolated from samples from donors subjected to different treatments (radiotherapy and chemotherapy), and in fibroblasts from donors not subjected to any treatment by between 50 and 68%, confirming its use for improving cell healing both in pathological situations which trigger an increase in Cx43 (such as ulcers of patients with diabetes for example), and in non-pathological situations (the decrease in Cx43 accelerates regeneration time and improves skin healing) (Ghatnekar *et al.*, 2009; Laird and Lampe, 2018; Ongstad *et al.*, 2013).

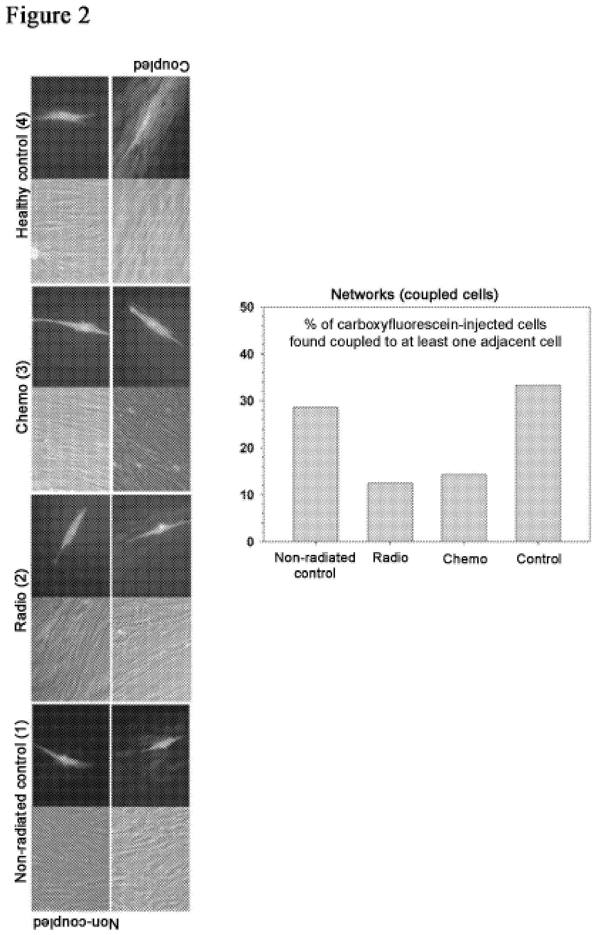

**Figure 2****.** Loss of intercellular communication capacity through gap junctions (GJs) in dermal fibroblasts of patients subjected to chemotherapy or radiotherapy. Cell coupling or dye coupling assay in which the transfer of 5,6-carboxyfluorescein (green) between fibroblasts in contact through GJs is studied. Fibroblasts isolated from patients with different subtypes of breast cancer and who have been treated with chemotherapy and/or radiotherapy in comparison with fibroblasts from patients with a benign pathology (not subjected to treatment) (healthy control) were used. Both chemotherapy and radiotherapy, despite increasing the levels of Cx43 in dermal fibroblasts, give rise to a lower cell coupling percentage with respect to the activity of GJs in fibroblasts from healthy donors. The graph depicts the percentage of fibroblasts coupled with adjacent fibroblasts through GJs and detected by the injection and transfer of 5,6-carboxyfluorescein.

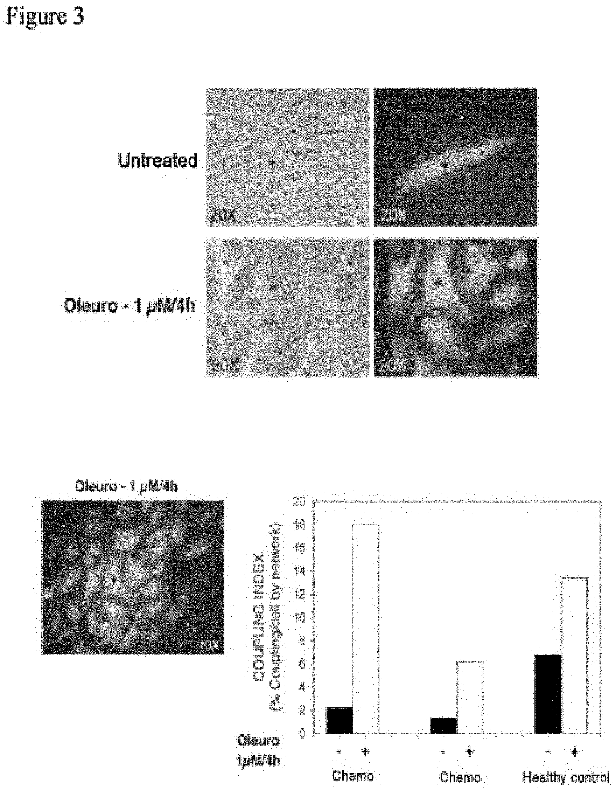

**Figure 3****.** Oleuropein restores intercellular communication through GJs and improves the cellular phenotype. Treatment of dermal fibroblasts with 1 µM oleuropein for 4 hours gave rise to a change in cellular morphology along with greater coupling through GJs, analyzed by means of the dye coupling technique. The asterisk (*) indicates the cell into which fluorophore 5,6-carboxyfluorescein (green) has been injected. The percentage of cells coupled through GJs is depicted in graph C.

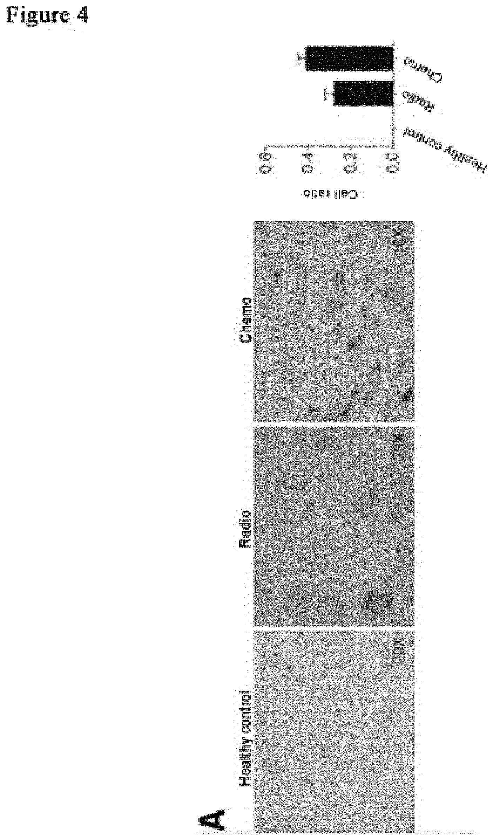

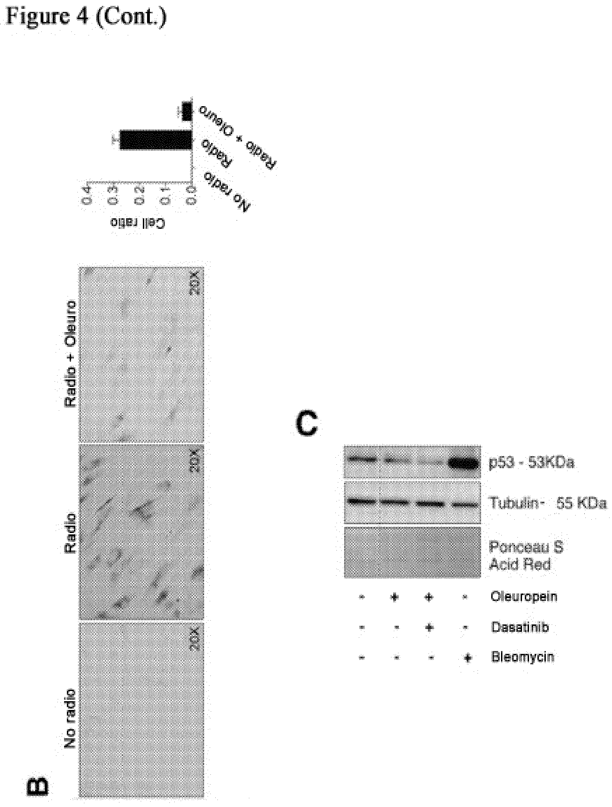

**Figure 4****.** The dermal fibroblasts isolated from the skin subjected to radiotherapy and/or chemotherapy show high levels of cellular senescence. A. In the paper published by Campisi *et. al.* (Demaria *et al.*, 2014), cells which activate cellular senescence during skin regeneration process were identified. These authors demonstrated that endothelial cells and fibroblasts, but not keratinocytes, undergo cellular senescence (Demaria *et al.*, 2014), and the accumulation of senescent cells or a defective elimination thereof triggers fibrosis (Mosteiro *et al.*, 2016; Munoz-Espin and Serrano, 2014). Chemotherapy and radiotherapy increase cellular senescence in comparison with fibroblasts isolated from the skin of healthy donors. To determine the level of cellular senescence, the blue X-Gal signal has been quantified using the β-galactosidase assay. The graph shows the ratio of senescent cells with an intense blue signal with respect to total cells (Radio; radiotherapy. Chemo; chemotherapy). B. Treatment with 10 µM of oleuropein for 72 hours after exposure to radiation reduced the percentage of senescence in dermal fibroblasts (originating from the skin of a healthy donor) subjected to experimental radiotherapy of 55 Grays for about 30 minutes, according to specifications of the linear accelerator model. The graph depicts the ratio of senescent cells (intense blue signal) with respect to total cells. C. Treatment of dermal fibroblasts exposed to radiation (55 Grays, 30 minutes) with 10 µM oleuropein for 48 hours reduced the levels of senescence factor p53 to levels similar to treatment with the senolytic dasatinib at 0.2 µM combined with 10 µM oleuropein. To induce cellular senescence, the fibroblasts were treated with 50 µg/µL of bleomycin. The immunoblot against protein p53, using as loading controls tubulin and Ponceau red stain (Ponceau S Acid Red) is shown.

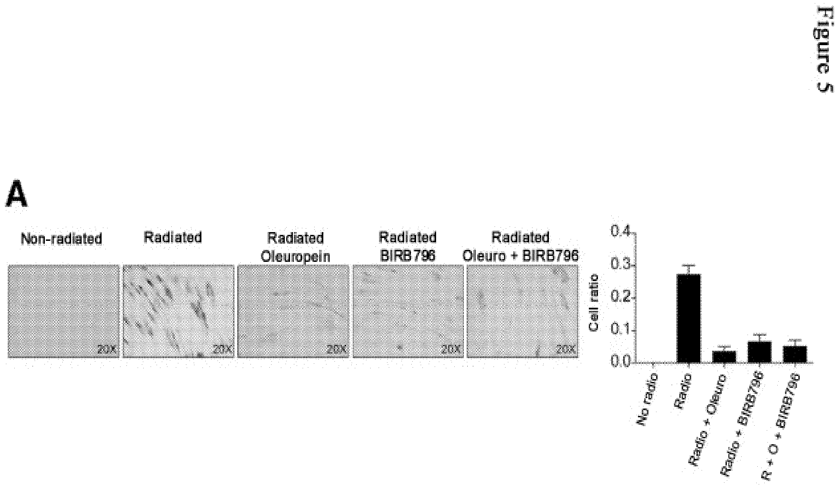

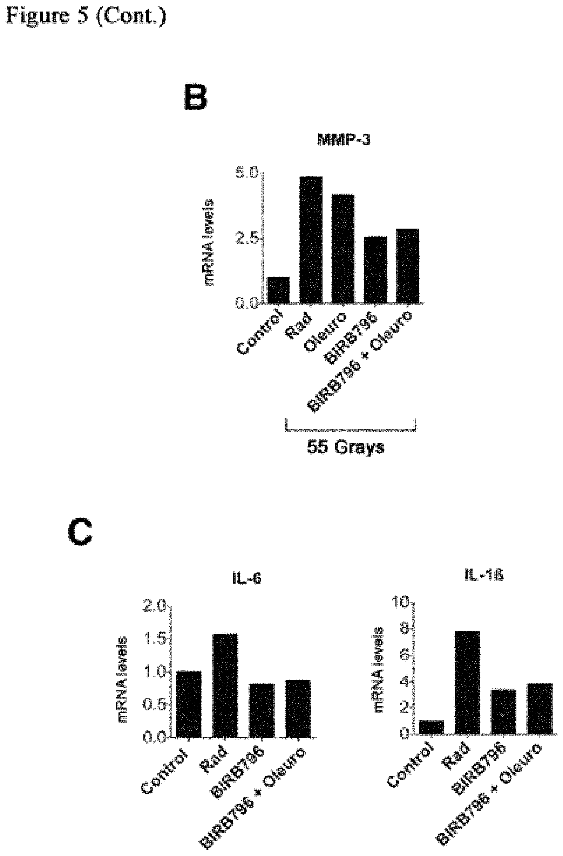

**Figure 5****.** Post-radiotherapy modulatory effect of oleuropein and BIRB796 in cellular senescence and senescence-associated secretory phenotype (SASP). A. Treatment with 10 µM oleuropein for 72 hours of fibroblasts isolated from healthy skin and subjected to experimental radiotherapy (55 Grays) was able to lower the accumulation of senescent cells. Its combination with SASP inhibitor BIRB796 (2.5 µM, 72 hours) did not modify the effect of oleuropein, with a similar reduction in the percentage of senescent cells being detected. Levels of cellular senescence have been quantified by way of the blue X-Gal signal (β-galactosidase assay). The graph shows the ratio of senescent cells with an intense blue signal with respect to total cells. B, C. The presence of senescent cells limits tissue regeneration capacity, and the accumulation thereof triggers fibrosis, as enzymes involved in remodeling the matrix, the inflammatory component, and factors such as IL-6, which participate in neighboring cell reprogramming, are synthesized and released (Mosteiro *et al.*, 2016; Mosteiro *et al.*, 2018). Treatment for 72 hours of fibroblasts isolated from healthy skin and subjected to experimental radiotherapy of 55 Grays with BIRB796 (2.5 µM) protects against the synthesis of SASP components such as MMP-3, which is involved in healing as it breaks down different types of collagens and fibronectin (B). Treatment with oleuropein would reduce the levels of cellular senescence (accumulation of senescent cells), and the combination thereof with BIRB796 would reduce the SASP component, thereby interfering with excess remodeling of the extracellular matrix (breakdown of collagens III, IV, IX, and X and other skin components). Moreover, the presence of BIRB796 decreases synthesis of the inflammatory component (IL-1β and IL-6) in the presence or in the absence of oleuropein (C), decreasing inflammation in the tissue damaged in the presence of senescent cells. MMP-3, IL-6, and IL-1β mRNA levels were normalized against control gene HPRT1 and depicted in comparison to a non-radiated healthy fibroblast control. It should be mentioned that BIRB796 is a new generation inhibitor of p38 MAPK, a molecule which is involved in the senescence process in fibroblasts and which, as a result of its efficacy and toxicological profile, has reached clinical trial phases II and III in inflammatory diseases such as rheumatoid arthritis (identifier at clinicaltrials.gov NCT02214888, Boehringer Ingelheim), Crohn's disease, ulcerative colitis, and psoriasis (identifier NCT02209753, Boehringer Ingelheim). (Chiacchiera *et al.*, 2012; Force *et al.*, 2004; He *et al.*, 2013; Ma *et al.*, 2014; Mosteiro *et al.*, 2018; Thalheimer *et al.*, 2014; Zhang *et al.*, 2014).

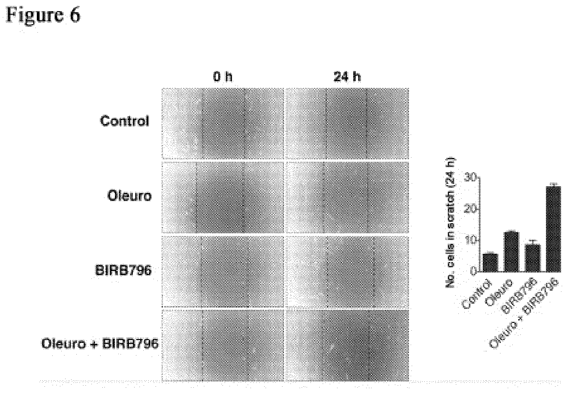

**Figure 6****.** Combined oleuropein-BIRB796 treatment favors dermal fibroblast migration in a wound healing or scratch assay. Scratch assay in dermal fibroblasts initially originating from healthy skin and experimentally radiated at 55 Grays, to which treatments with 10 µM oleuropein, 2.5 µM BIRB796, or the combination of both were applied for 24-48 hours. The state of the assay 24 hours after having made the scratch, as well as the quantification of total cells present in the original space of the scratch (demarcated by dotted lines) after this time slot for each of the conditions are shown. The wound healing assay is a direct and cost-effective method for studying *in vitro* cell migration. It is based on the fact that by making an artificial wound (referred to as scratch) in a confluent cell monolayer, cells from the recently made edges move towards the opening to close the scratch until establishing new cellular contacts. In general terms, a scratch is made in a cell monolayer, images are taken at the start and at regular intervals during the migration of the cells, and they are finally compared to determine the cell migration rate.

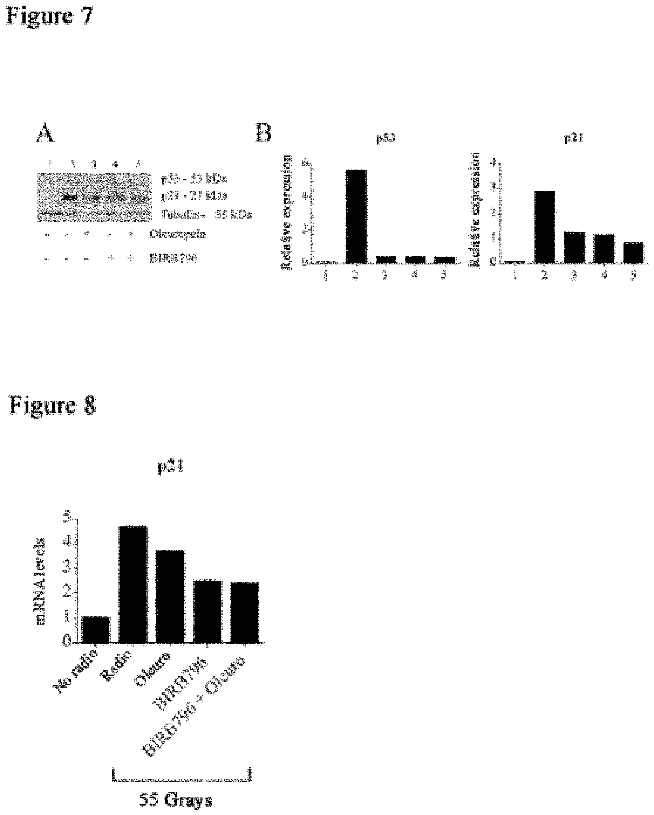

**Figure 7****.** Post-radiotherapy treatment with oleuropein and BIRB796 reduces the production of factor p53 and its target p21, which are involved in cellular senescence in dermal fibroblasts. A. Experimental radiation of fibroblasts isolated from healthy skin (sample 1) at 55 Grays (sample 2) involved an increase in senescence factors p53 and p21. Post-radiation treatment for 72 hours with 10 µM oleuropein (sample 3) or the SASP inhibitor BIRB796 2.5 µM (sample 4) effectively reduced protein levels of both factors, with the combination of both compounds (sample 5) being the most effective for reducing p21. B. Quantification of the protein levels of p53 and p21 of the Western blot shown in A relativized to the expression of the loading control, tubulin.
**Figure 8****.** Post-radiotherapy modulatory effect of oleuropein and BIRB796 on cellular senescence in dermal fibroblasts. Treatment for 72 hours of fibroblasts isolated from healthy skin and subjected to experimental radiotherapy of 55 Grays with oleuropein (10 µM), BIRB796 (2.5 µM), and the combination of both reduces the gene expression of senescence factor p21. The p21 mRNA level was normalized against control gene HPRT1 and depicted in comparison to a non-radiated healthy fibroblast control.

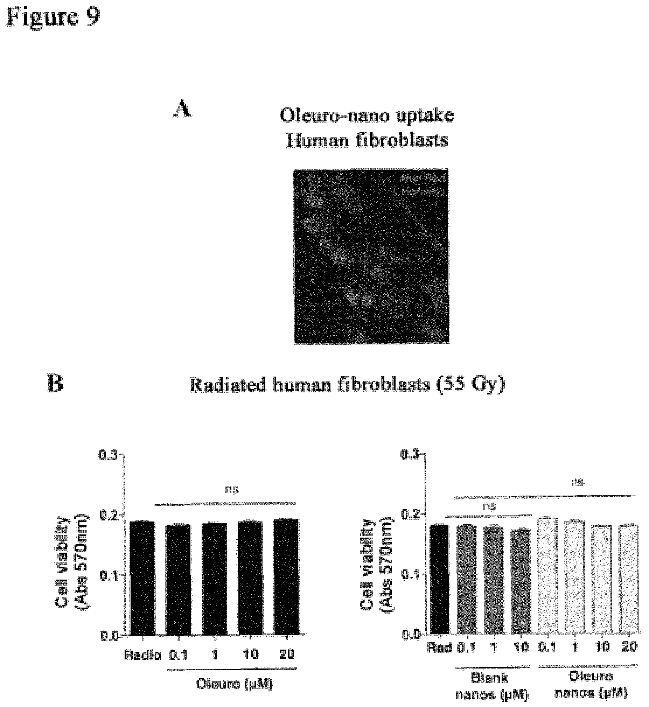

**Figure 9****.** Oleuropein nanocapsules. Dermal fibroblasts isolated from the skin were treated with encapsulated oleuropein (oleuro-nanos) and labeled with Nile Red. Encapsulation does not affect the capacity of oleuropein to cross the plasma membrane. The cell viability assay in radiated dermal fibroblasts (55 Gy) demonstrates that oleuro-nanos are not toxic for the cell.

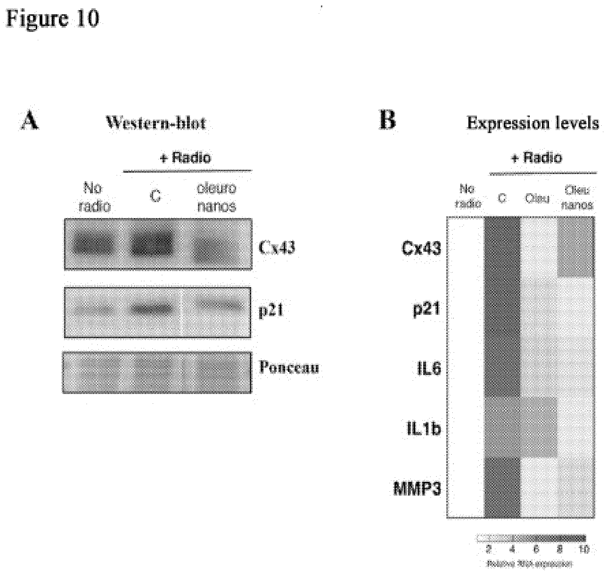

**Figure 10****.** Effect of oleuro-nanos on radiated dermal fibroblasts. Oleuro-nanos (10 µM) reduce the levels of Cx43 and p21 in radiated fibroblasts. Treatment for 72 hours of fibroblasts isolated from healthy skin and subjected to experimental radiotherapy of 55 Grays with oleuropein (10 µM) and oleuro-nanos (10 µM) reduces the expression levels of Cx43, p21, and SASP factors such as IL6, ILlb, and MMP3. RNA levels were normalized against control gene HPRT1 and depicted in comparison to a non-radiated healthy fibroblast control (no radio).

### Brief Description of the Invention

The present invention relates to the use of a composition for the treatment and/or prevention of the side effects of radiotherapy and/or chemotherapy, preferably as a protective post-treatment composition, that is, the effect is produced once the cells have been radiated (restores phenotype and gap junctions and reduces senescence). Accordingly, the composition comprises two chemical compounds that can protect and treat tissues that have been affected or are at risk of being affected by the side effects of chemotherapy and/or radiotherapy. The present invention also relates to a kit comprising the composition of the present invention for the treatment and/or prevention of the side effects of radiotherapy and/or chemotherapy.

### Detailed Description of the Invention

### Definitions

Unless specifically mentioned otherwise, in the context of this application, the term *"comprises"* is used to indicate that the list of components can include optional aspects that may or may not be mentioned. The term *"comprises"* can also include the concept *"consists of".*

The terms *"treatment"* or *"therapy"* as they are used herein refer to a set of hygienic, pharmacological, surgical, and/or physical means, the purpose of which is to cure or relieve diseases and/or symptoms. The terms *"treatment"* or *"therapy"* comprise both prophylactic and curative methods, since they are both aimed at maintaining or restoring health. Regardless of the source of the illness, disease, or disability, the relieve thereof by means of the administration of a suitable remedy is understood as therapy or therapeutic use in the context of the present invention.

The term *"prevention"* as it is used herein refers to a set of hygienic, pharmacological, surgical, and/or physical means the purpose of which is to prevent diseases and/or symptoms from developing in an animal or individual. The term *"prevention"* comprises prophylactic methods since it is aimed at maintaining health.

The term *"radiotherapy"* as it is used herein refers to a form of treatment based on the use of ionizing radiations. Said ionizing radiations comprise x-rays or radioactivity, which includes gamma rays and alpha particles.

The term *"chemotherapy"* as it is used herein refers to a form of treatment based on the administration of chemical substances for the treatment of a disease, particularly cancer. The term *"chemotherapy"* also refers to a form of treatment based on the administration of therapeutic antibodies and/or drugs to treat an individual or animal that suffers or is at the risk of suffering cancer.

In the present invention, radiotherapy and/or chemotherapy can be part of a neoadjuvant therapy.

The term *"antibody"* as it is used herein refers to immunoglobulin molecules and immunologically active fragments of immunoglobulin molecules, that is, molecules which contain an antigen binding site that binds specifically (immunoreacts). Examples of immunologically active fragments include F(ab) and F(ab')₂ fragments which can be generated by treating the antibody with an enzyme such as pepsin. The antibodies can be polyclonal (they typically include different antibodies targeting different determinants or epitopes) or monoclonal (targeting a single determinant in the antigen).

The monoclonal antibody can be altered biochemically or by means of genetic manipulation, or it can be synthetic, the antibody possibly lacking, entirely or in parts, fragments or portions that are not required for antigen recognition and being substituted by others conferring additional advantageous properties to the antibody.

The antibody can also be recombinant, chimeric, humanized, synthetic, or a combination of any of the foregoing. A *"recombinant antibody or polypeptide"* (rAB) is an antibody that has been produced in a host cell which has been transformed or transfected with the nucleic acid which encodes the polypeptide or antigen, or produces the polypeptide or antigen as a result of homologous recombination.

The term *"therapeutic antibodies"* as it is used herein refers to a medicinal product for the treatment of cancer which comprises but is not limited to: trastuzumab, rituximab, gemtuzumab ozogamicin, alemtuzumab, ibritumomab tiuxetan, tositumomab-I-131, cetuximab, bevacizumab, panitumumab, ofatumumab, ipilimumab, brentuximab vedotin, pertuzumab, ado-trastuzumab emtansine, and/or obinutuzumab.

The term "drugs" as it is used herein refers to a medicinal product for the treatment of diseases, such as cancer, for example, and comprises but is not limited to: abiraterone, altretamine, anhydrovinblastine, auristatin, bexarotene, bicalutamide, BMS 184476, 2,3,4,5,6-pentafluoro-N-(3-fluoro-4-methoxyphenol)benzene sulfonamide, bleomycin, N,N-dimethyl-L-valyl-L-valyl-N-methyl-L-valyl-L-proly-1-L-proline-t-butylamide, cachectin, cemadotin, chlorambucil, cyclophosphamide, 3',4'-didehydro-4'-deoxy-8'-Norvin-caleukoblastine, docetaxol, doxetaxel, carboplatin, carmustine, cisplatin, cryptophycin, cytarabine, dacarbazine (DTIC), dactinomycin, daunorubicin, decitabine dolastatin, doxorubicin (adriamycin), etoposide, 5-fluorouracil, finasteride, flutamide, hydroxyurea, and hydroxyurea taxanes, ifosfamide, liarozole, lonidamine, lomustine (CCNU), MDV3100, mechlorethamine, melphalan, mivobulin isethionate, rhizoxin, sertenef, streptozocin, mitomycin, methotrexate, taxanes, nilutamide, onapristone, paclitaxel, prednimustine, procarbazine, RPR109881, estramustine phosphate, tamoxifen, tasonermin, taxol, tretinoin, vinblastine, vincristine, vindesine sulfate, and/or vinflunine.

The term *"neoadjuvant therapy"* as it is used herein refers to a treatment which is administered as a first step for reducing the size of the tumor before the primary treatment which generally consists of surgery. Chemotherapy, radiotherapy, and hormone therapy are among the examples of adjuvant therapy.

The terms *"side effect"* or *"adverse reaction to medicinal products"* as they are used herein refer to any response to a medicinal product which is harmful and unintentional, and which takes place at doses that are normally applied in animals or individuals for the prophylaxis, diagnosis, or treatment of diseases and/or symptoms.

The terms *"individual", "patient",* or *"subject"* are used interchangeably herein and do not intend to be limiting in any way, where the *"individual", "patient",* or *"subject"* may be of any age, sex, and physical condition. The term *"animal"* as it is used herein refers to any eukaryotic, heterotrophic, and pluricellular non-human organism.

The term *"therapeutically effective amount"* refers to an amount of chemical substance or chemical compound in the composition which has therapeutic effect and is capable of relieving or curing the side effects of radiotherapy and/or chemotherapy.

### Active component

In a first aspect, the present invention provides a composition (hereinafter composition of the invention) for use in a method for the treatment and/or prevention of the side effects of radiotherapy and/or chemotherapy, preferably as a protective post-treatment composition. In a preferred embodiment, the composition comprises a chemical compound according to formula (I): 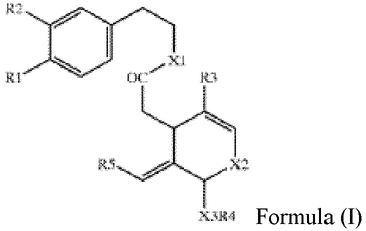 wherein
R1-R2 are, independently, -OH, -NH₂, or -SH; preferably R1 and R2 are -OH;
R3 and R5 are, independently, hydrogen, C1-C6 alkyl, C2-C6 alkenyl, C2-C6 alkynyl, aryl, -OH, C1-C6 alkoxy, halogen, NO₂, NH₃, or COOCH₃; preferably R3 is COOCH₃ and R5 is hydrogen;
XI -X3 are, independently, oxygen, sulfur, -CH₂, or carboxy; preferably X1, X2, and X3
R4 is hydrogen, C1-C6 alkoxy, glucose, β-D-glucopyranose, C1-C6 alkyl, C2-C6 alkenyl, C2-C6 alkynyl, aryl, -OH, halogen, NO₂, NH₃, carbohydrate, amino acid, nucleotide, lipid, or the enantiomer thereof; preferably R4 is glucose, or the enantiomer thereof.

In an alternative aspect relative to the first aspect, the present invention provides a composition for use in a method for the treatment and/or prevention of the side effects of radiotherapy and/or chemotherapy, preferably as a protective post-treatment composition following treatment with radiotherapy and/or chemotherapy, wherein said composition comprises a chemical compound according to formula (II) (compound BIRB 796), 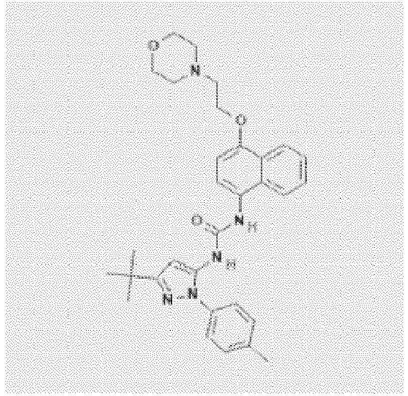 or an analog to the compound of formula II comprising the following chemical structure: 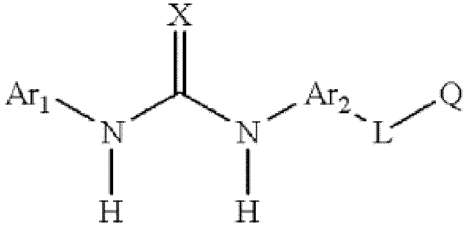 wherein so as to not unnecessarily increase the number of pages of this document, explicit reference is made to the definition given for each of the radicals Ar1, X, Ar2, L, and Q shown in the formula above, as set forth in column 6, line 48, to column 10, line 33 of patent document US6319921B1. Additionally, as specific examples of analogs of BIRB 796, the compounds expressly mentioned from column 10, line 55, to column 17, line 1 of patent document US6319921B1 are incorporated herein by explicit reference.

Additionally, the present invention also includes analogs of BIRB 796 such as those shown in patent US6319921B1, which document is incorporated herein in its entirety by reference.

The term *"active component"* as it is used herein refers to a chemical compound according to formula (I) and/or (II) and/or an analog of the compound of formula II as described above. It is hereby noted that, in a preferred manner and imbued with any aspect or embodiment of the present invention, said active component (I) can be combined with BIRB 796 (doramapimod) or an analog thereof in one and the same composition or in a "kit of parts" such that the administration thereof can be done in a simultaneous or sequential manner or in any order.

The *active component* of the composition of the present invention is thereby selected from a chemical compound according to formula (I) or (II) or an analog of the compound of formula II, or any combination thereof.

In a preferred embodiment, the composition comprises a plant extract containing a therapeutically effective amount of the chemical compound according to formula (I) optionally combined with a compound of formula (II) and/or an analog of the compound of formula II. The extract may comprise any extract obtained by means of an ethanol, acetone, inorganic and organic acid or base, and/or an aqueous solution extraction of any plant tissue, wherein a therapeutically effective amount of the chemical compound according to formula (I) can be extracted. In a preferred embodiment, the composition comprises an extract derived from *Olea europaea, Ligustrum obtusifolium, Fraxinus excelsior, F. angustifolia, F. chinensis, Syringa josikaea, S. vulgaris, Phillyrea latifolia, Ligustrum ovalifolium, L. vulgare,* or from any other genus belonging to the *Oleaceae* family.

In another preferred embodiment, the chemical compound according to formula (I) is oleuropein. Oleuropein is a chemical compound according to formula (III):

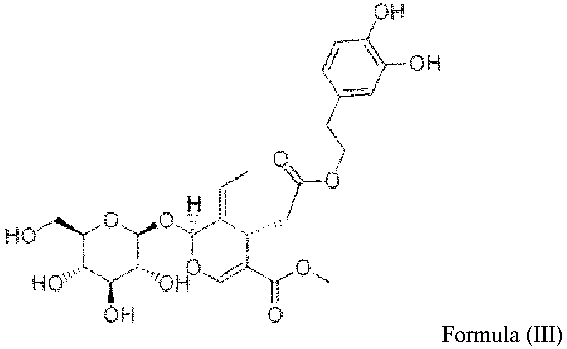

The composition of the present invention is used in a method for treating and/or preventing the side effects of radiotherapy and/or chemotherapy in the skin of an individual or animal. For example, but without limitation, the composition is used in a method for treating the side effects derived from treatment with radiotherapy and/or chemotherapy, preferably side effects in the skin such as radiation-induced fibrosis, inflammation, dermatitis, skin fragility, erosions in skin folds, loss of nails, or dry skin, caused by radiotherapy and/or chemotherapy.

In a preferred embodiment, the individual or animal suffers or is at risk of suffering cancer. In a preferred embodiment, the cancer is selected from a group comprising: prostate cancer, breast cancer, ovarian cancer, endometrial cancer, cervical cancer, lymphoid cancer, brain cancer, head and neck cancer, leukemia, lung cancer, colorectal cancer, thyroid cancer, renal cancer, adrenal cancer, liver cancer, stomach cancer, intestinal cancer, kidney cancer, multiple myeloma, non-small cell lung cancer, pancreatic cancer, glioblastoma, melanoma, colon cancer, lymphomas, and/or neuroendocrine tumors. Preferably, the cancer or tumor is breast cancer, brain cancer, head and neck cancer, and lymphomas. Even more preferably, the cancer or tumor is breast cancer.

### Additives

In a preferred embodiment, the composition further comprises one or more additives, such as a carrier solvent, a preservative, a surfactant, a gelling agent, and/or a pH buffer.

A *"carrier solvent"* refers to the component of the composition in which the active component is dissolved. The carrier solvent of the present invention may include, but is not limited to, vaseline, water, lanolin, polyethylene glycol, alcohols, and/or vegetable oils.

A *"preservative"* refers to a substance which stops or minimizes deterioration caused by the presence of different types of microorganisms. The preservative of the present invention may include, but is not limited to, methylparaben, propylparaben, benzyl alcohol, potassium sorbate, ethylhexylglycerin, phenoxyethanol, EDTA, grapefruit seed extract, tea tree oil, sodium benzoate, and/or dehydroacetic acid.

A *"surfactant"* refers to a substance which affects, by means of the surface tension, the contact surface between two phases. The surfactant of the present invention may include, but is not limited to, sodium lauryl sulfate, ammonium lauryl sulfate, disodium lauryl sulfosuccinate, cocoamphocarboxyglycinate, cocoaminopropyl betaine, and/or alpha olefin sulfonate.

A *"gelling agent"* refers to a substance which can gel the liquid in which it is dissolved. The gelling agent of the present invention may include, but is not limited to, agar-agar, alginine, carrageenan, collagen, corn starch, gelatin, guar gum, locust bean gum, pectin, and/or xanthan gum.

The pH buffer may include, but is not limited to, citric acid, lactic acid, sodium lactate, sodium acetate, sodium phosphate, sodium citrate, sodium borate, and/or sodium gluconate.

In a preferred embodiment, the composition can further comprise phospholipids, amino acids, vitamins, and/or peptides. The phospholipids, amino acids, vitamins, and/or peptides can help to protect, hydrate, and/or regenerate skin.

### Administration

In a preferred embodiment, the composition is administered by means of a topical release system. The composition of the present invention can be a gel, cream, or ointment. Said composition can be administered on the area affected by the side effects of radiotherapy and/or chemotherapy. Said composition can also be administered in a preventive manner on the area at risk of being affected by the side effects of radiotherapy and/or chemotherapy. For example, but without limitation, the composition can be applied on the skin affected or at risk of being affected by the side effects of radiotherapy and/or chemotherapy and can be gently rubbed in for several minutes so that the skin can absorb the active component.

In a preferred embodiment, the composition is administered immediately after a radiotherapy and/or chemotherapy session or one or two days after the session. In another preferred embodiment, the composition is used for treating the side effects of radiotherapy and/or chemotherapy 1-4 weeks after the treatment with radiotherapy and/or chemotherapy, and in another even more preferred embodiment, the composition is used to reduce the scars that were formed as a result of the side effects of radiotherapy and/or chemotherapy.

The composition of the present invention can be administered one or more times. A dose of the composition may comprise 0.5-1000 mg of the active component. Preferably, a dose comprises 0.5-100 mg, 0.5-50 mg, 0.5-25 mg, 0.5-10 mg, 0.5-2.5 mg, 1.0-2.5 mg, or 1.5-2.5 mg of active component. In a preferred embodiment, the composition contains a therapeutically effective amount of the active component of the present invention.

### Kit

In a second aspect, the present invention provides a kit for use after the treatment and/or prevention of the side effects of radiotherapy and/or chemotherapy in an animal or individual, wherein said kit comprises the composition of the present invention, as described above. It should be noted that said kit could be configured as a kit of parts, containing in separate containers a composition which comprises as an active ingredient the compound of formula (I), and a composition which comprises the compound of formula (II) and/or an analog of the compound of formula II.

In a preferred embodiment, the composition of the kit comprises a plant extract containing a therapeutically effective amount of the chemical compound according to formula (I) and optionally a compound of formula (II) and/or an analog of the compound of formula II. The extract may comprise any extract obtained by means of an ethanol, acetone, inorganic and organic acid or base, and/or aqueous solution extraction of any plant tissue, wherein a therapeutically effective amount of the chemical compound according to formula (I) can be extracted. In a preferred embodiment, the composition of the kit comprises an extract derived from *Olea europaea, Ligustrum obtusifolium,* etc. In another preferred embodiment, the chemical compound according to formula (I) is oleuropein (formula III).

The kit of the present invention is used in a method for treating and/or preventing the side effects of radiotherapy and/or chemotherapy in the skin of an individual or animal. For example, but without limitation, the kit is used in a method for treating and/or preventing the side effects derived from treatment with radiotherapy and/or chemotherapy, preferably side effects in the skin such as radiation-induced fibrosis, inflammation, dermatitis, skin fragility, erosions in the skin folds, loss of nails, or dry skin, caused by radiotherapy and/or chemotherapy.

### Examples

### Primary fibroblast isolation, cell culture, and treatments

Skin tissue biopsies from female patients of A Coruña University Hospital Complex - A Coruña Integrated Management Services (*Complejo Hospitalario Universitario de A Coruña* - *Xerencia Xestión Integrada A Coruña* (CHUAC-XXIAC)) were obtained from operations for the surgical removal of a benign pathology (fibroadenoma, samples consisting of untreated healthy skin) and from oncology interventions (samples consisting of skin subjected to radiotherapy and chemotherapy). Signed informed consent was received from each of the patients (2015/029, C.0003333, 2016/077). To isolate primary fibroblast from these samples, hypodermal fat was removed with the aid of a scalpel, and the epidermis was segmented into fragments of about 1x1 cm (explants). Lines were made with a scalpel on a 100 cm² adherent Petri dish creating a grid, and each of the explants was placed in the resulting intersections with the epidermis facing up and the dermis in contact with the dish. After incubating for several minutes in the cell culture oven at 37°C to favor adhesion, Dulbecco's Modified Eagle Medium (DMEM, Invitrogen Life Technologies) supplemented with 20% fetal bovine serum and 1% penicillin/streptomycin (100 U/mL of penicillin, 100 µg/mL of streptomycin; Gibco, Invitrogen Corporation) was added and they were left in the cell culture oven at 37°C, 5% CO₂, and moisture saturation with periodic changes of the medium until primary fibroblasts were observed. Upon reaching a confluence of 70%, the explants were discarded and cells were transferred to another culture dish, regularly expanding said cells in DMEM with 10% fetal bovine serum and 1% penicillin/streptomycin. Healthy dermal primary fibroblasts and fibroblasts originating from skin subjected to radiotherapy and chemotherapy in intermediate culture passages (passages 5 to 10) were used in the experiments. They were subjected to treatments with different molecules in order to observe the effect thereof on connexin43 (Cx43) and the cellular senescence process: oleuropein (Extrasynthese), the senolytic agent dasatinib (Bristol-Myers Squibb Pharma EEIG), the senescence inducing agent bleomycin (Almirall^{®}), and the SASP inhibitor BIRB796 (MedChemExpress). The working concentrations of said compounds were determined by means of literature review and cell viability assays (MTT).

### Fibroblast radiation in primary culture

To simulate radiotherapy effects on healthy dermal fibroblasts, experimental radiotherapy tests in which cells were exposed to 55 Grays in the Varian TrueBeam linear electron accelerator (Radiotherapy Department of the Cancer Center of Galicia) were carried out. To that end, the cells were transported in sealed cell culture supports with 1 cm thick culture medium to ensure a uniform radiation, and this medium was renewed 5 hours after performing the radiation.

### Immunofluorescence

Dermal fibroblasts in primary cultures were fixed with 2% paraformaldehyde in PBS. After washing with PBS, they were incubated with a 0.1 M glycine solution for 10 minutes at room temperature. The membranes were permeabilized with a 0.2% Triton X-100 solution in PBS for 10 minutes at room temperature. To block the non-specific binding sites in the preparation, the cells were incubated for 30 minutes with a 1% bovine serum albumin solution in PBS supplemented with 0.1% Tween-20 (PBS-T). The cells were then incubated with primary rabbit anti-Cx43 antibody (C6219, Sigma-Aldrich) for 1 hour at room temperature. After washing excess primary antibody with PBS-T, secondary goat anti-rabbit antibody labeled with fluorescein isothiocyanate (FITC) (F2765, Invitrogen) was incubated for 1 hour at room temperature and in darkness. Excess secondary antibody was removed with PBS-T and nuclei were stained with 4',6-diamino-2-phenylindole (DAPI) for 4 minutes at room temperature and in darkness. Lastly, excess DAPI was washed and the preparations were mounted with glycergel for subsequent analysis under the microscope.

### Western Blot

Fibroblast cell lysis was carried out in an ice-cold buffer (150 mM NaCl; 50 mM Tris-HCl, 5 mM EDTA, 0.5% NP-40, 0.1% SDS, 0.5% Sarkosyl). Equal amounts of proteins were split into a denaturing 10% acrylamide/bis-acrylamide gel with SDS and transferred to a poly(vinylidene fluoride) membrane in a Trans-Blot^{®} SD Semi-Dry Transfer Cell. After blocking the non-specific binding of the membrane by means of a 1-hour incubation at room temperature with a 5% milk solution in TBS-T buffer (20 mM Tris, 150 mM NaCl, 0.05% Tween-20), the membranes were incubated with the corresponding primary antibody for 16 hours at 4°C under rotation. Excess antibody was removed by means of successive washing with TBS-T buffer, after which it was incubated with secondary antibody labeled with horseradish peroxidase (HRP) for 1 hour at room temperature under rotation. After removing excess secondary antibody by means of washing with TBS-T, the signal was visualized by means of chemiluminescence in an Amersham Imager 600 developing chamber. Primary mouse anti-tubulin antibodies (T9026, Sigma-Aldrich), primary rabbit anti-Cx43 antibodies (C6219, Sigma-Aldrich), primary mouse anti-p53 antibodies (sc-126, Santa Cruz Biotechnology), primary mouse anti-p21 antibodies (sc-6246, Santa Cruz Biotechnology) and primary mouse anti-GAPDH antibodies (6C5, Thermo Fisher Scientific), and secondary sheep anti-mouse antibodies (NA-931, Sigma-Aldrich) and secondary goat anti-rabbit antibodies (A6154, Sigma-Aldrich), were used.

### Cell coupling by means of dye coupling

Fibroblasts were cultured to a confluence of about 70%. A 5,6-carboxyfluorescein solution was injected into a cell and the transfer of this fluorophore to adjacent cells was evaluated.

### Hematoxylin staining for phenotype determination

To study the phenotype of the different cell types, fibroblasts were cultured on glass coverslips previously treated with poly-D-lysine hydrobromide (Sigma) to ensure adherence, until reaching a confluence of 80%. They were then fixed with 4% paraformaldehyde and washed with phosphate-buffered saline (PBS). Staining was performed with Gill III hematoxylin for 5 minutes. Dehydration was performed by means of passages in increasing concentrations of alcohol (80° - 90° - 100°) and finally in xylene. They were mounted on slides and observed and photographed under an Olympus BX61 microscope.

### β-galactosidase senescence assay

The degree of β-galactosidase senescence and activity of the samples was determined by means of analyzing blue X-Gal staining using Senescence Cells Histochemical Staining Kit (Sigma). The protocol specified by the manufacturer was followed. Briefly, cells were cultured on glass coverslips previously treated with poly-D-lysine hydrobromide, fixed, and incubated overnight in the staining solution in an oven at 37°C in the absence of CO₂. Finally, they were mounted and observed and photographed under an Olympus BX61 microscope. The counting of cells positive for X-Gal, being differentiated between moderate staining and intense staining, was performed and the ratio thereof with respect to the total cells was counted. The ImageJ (NIH) image analysis software was used.

### Gene expression analysis (qPCR)

For RNA extraction from the different cell types, TRI Reagent (MRC) was used following the manufacturer's instructions. Once isolated, RNA was subjected to treatment with DNAse to remove the possible contaminating DNA residues (1 µL of DNAse, 2 µL of 10X reaction buffer - 200 mM Tris-HCL, pH 8.4, 20 mM MgCl₂, 500 mM KCl - and completing with water up to 20 µL). To that end, the reaction was incubated at 37°C for 15 minutes and finally stopped with 25 mM of ethylenediaminetetraacetic acid (EDTA) pH 8 for 15 minutes at 65°C. The amount and quality of the obtained RNA was quantified in Nanodrop ND-1000. Complementary DNA (cDNA) was obtained from the RNA extracted in the preceding step using the Superscript^{®} VILOTM cDNA Synthesis Kit (Invitrogen), with the reverse transcription of 1 µg of RNA. The reverse transcription program comprised the following stages: 10 minutes at 25°C, 60 minutes at 42°C, 5 minutes at 85°C, and maintaining the samples at 4°C. Finally, the obtained DNA was amplified using PowerUp^{™} SYBR^{®} Green Master Mix (Thermo Fisher Scientific) in the LightCycler 480 Instrument from Roche Applied Science. Replicates of each condition were performed using specific sense and antisense primers for the genes of interest: hypoxanthinephosphoribosyltransferase 1 (HPRT1), interleukin-1β (IL-1β), interleukin-6 (IL-6), p21, and matrix metalloproteinase-3 (MMP-3). The reaction program consisted of the following stages: incubation at 95°C for 10 minutes, 50-cycle amplification at 95°C for 10 seconds and at 65°C for 1 minute, and cooling for 20 seconds until 4°C. HPRT1 was used as the reference gene, and gene expression levels were analyzed relative to a cell control (non-radiated or radiated cells not subjected to any treatment).

The primers used are described in detail below: HPRT1: TTGAGTTTGGAAACATCTGGAG (sense), GCCCAAAGGGAACTGATAGTC (antisense). IL-Iβ: CGAATCTCCGACCACCACTAC (sense), TCCATGGCCACAACAACTGA (antisense). IL-6: TGTAGCCGCCCCACACA (sense), GGATGTACCGAATTTGTTTGTA (antisense). MMP-3: CCCTGGGTCTCTTTCACTCA (sense), GCTGACAGCATCAAAGGACA (antisense)

### Cell viability

Fibroblasts subjected to experimental radiotherapy of 55 Grays were cultured until reaching a confluence of 80%. They were then treated for 72 hours with different concentrations of oleuropein, empty nanoparticles, and nanoparticles with encapsulated oleuropein. After the end of treatments, cells were washed with PBS, fixed with 4% paraformaldehyde in PBS for 10 minutes, and stained with a cresyl violet acetate solution (Sigma) for 10 minutes. They were washed twice with PBS and left to dry. Lastly, cresyl violet was dissolved with a 30% acetic acid solution in distilled water for 15 minutes, taking 100 µL of each sample in duplicate and measuring absorbance at 570 nm in a Nano Quant Infinite M200 reader.

### Nanoparticle uptake in radiated fibroblasts

Nanoparticles labeled with Nile Red that are empty and loaded with different concentrations of oleuropein were added, diluted in a culture medium, to radiated fibroblasts in culture. After 4 hours, cell nuclei were stained with Hoechst (Invitrogen) diluted in culture medium at 100 ng/mL for 45 minutes and cells were photographed by means of a Leica TCS SP8 confocal fluorescence microscope.

### Wound healing or scratch assay

Fibroblasts were seeded in 6-well culture plates and left to grow until reaching a confluence of 90 - 100%. At that time, a wound or scratch was made in the center of the well with the aid of the tip of a 100 µL pipette, with the longitudinal axis always being perpendicular to the bottom of the well. Each well was then carefully washed with culture medium (DMEM with 10% fetal bovine serum and 1% penicillin/streptomycin) to remove cells that may have become detached, and finally experimental treatments were applied in DMEM with 10% fetal bovine serum and 1% penicillin/streptomycin supplemented with the different compounds object of the study. Each well was photographed under a microscope on the day the scratch was made and every 24 hours until the end of the experiment. The total cells present in the original space of the wound were quantified for each condition.

## Claims

1. A composition comprising a chemical compound according to formula (I): 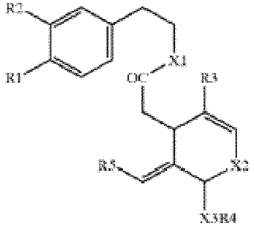 wherein R1-R2 are, independently, -OH, -NH₂, or -SH;
R3 and R5 are, independently, hydrogen, C1-C6 alkyl, C2-C6 alkenyl, C2-C6 alkynyl, aryl, -OH, C1-C6 alkoxy, halogen, NO₂, NH₃, or COOCH₃;
X1-X3 are, independently, oxygen, sulfur, -CH₂, or carboxy; and
R4 is hydrogen, C1-C6 alkoxy, glucose, β-D-glucopyranose, C1-C6 alkyl, C2-C6 alkenyl, C2-C6 alkynyl, aryl, -OH, halogen, NO₂, NH₃, carbohydrate, amino acid, nucleotide, lipid, or the enantiomer thereof
for use in the treatment of the side effects of radiotherapy and/or chemotherapy in an animal or individual, in combination, in a simultaneous or sequential manner, with BIRB 796 (doramapimod).

2. The composition for use according to claim 1, **characterized in that** the chemical compound according to formula (I) is oleuropein.

3. The composition for use according to any of claims 1 to 2, **characterized in that** the side effects of radiotherapy and/or chemotherapy present in the skin of an animal or individual.

4. The composition for use according to any of claims 1 to 3, **characterized in that** the individual or animal suffers cancer.

5. The composition for use according to any of claims 1 to 4, **characterized in that** the individual or animal suffers breast cancer.

6. The composition for use according to any of claims 1 to 5, **characterized in that** the composition comprises a carrier solvent, a preservative, a surfactant, a gelling agent and/or a pH buffer.

7. The composition for use according to any of claims 1 to 6, **characterized in that** the composition comprises phospholipids, amino acids, vitamins, and/or peptides.

8. The composition for use according to any of claims 1 to 7, **characterized in that** the composition is a gel, cream, or ointment.

9. Use of a kit in the preparation of a medicinal product for the treatment and/or prevention of the side effects of radiotherapy and/or chemotherapy in an animal or individual, **characterized in that** the kit comprises a composition which in turn comprises a chemical compound according to formula (I): 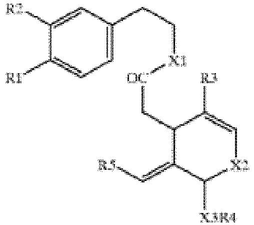 wherein R1-R2 are, independently, -OH, -NH₂, or -SH;
R3 and R5 are, independently, hydrogen, C1-C6 alkyl, C2-C6 alkenyl, C2-C6 alkynyl, aryl, -OH, C1-C6 alkoxy, halogen, NO₂, NH₃, or COOCH₃; XI -X3 are, independently, oxygen, sulfur, -CH₂, or carboxy; and
R4 is hydrogen, C1-C6 alkoxy, glucose, β-D-glucopyranose, C1-C6 alkyl, C2-C6 alkenyl, C2-C6 alkynyl, aryl, -OH, halogen, NO₂, NH₃, carbohydrate, amino acid, nucleotide, lipid, or the enantiomer thereof; and
wherein the kit further comprises BIRB 796 (doramapimod).

10. Use according to claim 9, **characterized in that** the chemical compound according to formula (I) is oleuropein.

11. Use according to any of claims 9 to 10, **characterized in that** the side effects of radiotherapy and/or chemotherapy present in the skin of an animal or individual.

12. Use according to any of claims 9 to 11, **characterized in that** the individual or animal suffers cancer.

13. Use according to any of claims 9 to 12, **characterized in that** the individual or animal suffers breast cancer.
